(19) Europäisches Patentamt
European Patent Office
Office européen des brevets

(11) Veröffentlichungsnummer: **0 191 444**
**A2**

# (12) EUROPÄISCHE PATENTANMELDUNG

(21) Anmeldenummer: **86101661.6**

(22) Anmeldetag: **10.02.86**

(51) Int. Cl.⁴: **C 07 D 249/08**, A 01 N 43/653

(30) Priorität: **13.02.85 DE 3504897**

(43) Veröffentlichungstag der Anmeldung: **20.08.86 Patentblatt 86/34**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Buschmann, Ernst, Dr., Georg-Ludwig-Krebs-Strasse 10, D-6700 Ludwigshafen (DE)**
Erfinder: **Sproesser, Linhard, Dr., Goethestrasse 4, D-6702 Bad Dürkheim (DE)**
Erfinder: **Zeeh, Bernd, Dr., Queichstrasse 5, D-6703 Limburgerhof (DE)**
Erfinder: **Jung, Johann, Prof.Dr., Hardenburgstrasse 19, D-6703 Limburgerhof (DE)**
Erfinder: **Rademacher, Wilhelm, Dr., Austrasse 1, D-6703 Limburgerhof (DE)**
Erfinder: **Ammermann, Eberhard, Dr., Sachsenstrasse 7, D-6700 Ludwigshafen (DE)**
Erfinder: **Pommer, Ernst-Heinrich, Dr., Berliner Platz 7, D-6703 Limburgerhof (DE)**

(54) **Diastereomere Triazolylglykolether - Fungizide und Wachstumsregulatoren.**

(57) Diastereomere der Formel

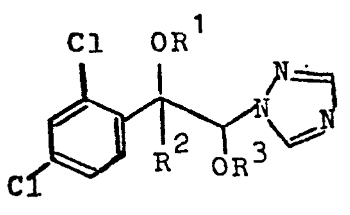

I,

in der
$R^1$ für Wasserstoff, Alkyl, Alkenyl, Alkinyl und Benzyl,
$R^2$ für Alkyl und Alkenyl,
$R_3$ für Alkyl und Benzyl steht, wobei die asymmetrischen Zentren des Moleküls a) RR oder SS oder b) RS oder SR konfiguriert sind und Verfahren zu ihrer Herstellung sowie die Verbindungen enthaltende Fungizide.

EP 0 191 444 A2

0 191 444

Diastereomere Triazolylglykolether - Fungizide und Wachstumsregulatoren

Die vorliegende Erfindung betrifft neue Diastereomere von Triazolylglykolethern, Verfahren zu ihrer Herstellung und die Verwendung der Substanzen zur Bekämpfung pathogener Pilze und zur Regulierung des Pflanzenwachstums.

Es ist bekannt, daß Triazolylglykolether pflanzenwachstumsregulierende und fungizide Eigenschaften besitzen (DE-OS 29 26 280, 30 47 726, 31 50 204). Die Wirksamkeit der Produkte ist gut, jedoch ist der bei niedrigen Aufwandmengen erzielte Effekt nicht befriedigend.

Es wurden nun Diastereomere der Triazolylglykolether der Formel

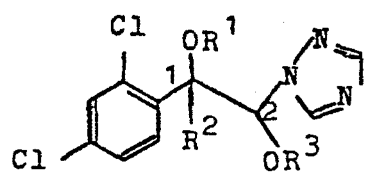

I

gefunden, in der

$R^1$ Wasserstoff, Alkyl, Alkenyl, Alkinyl oder gegebenenfalls substituiertes Benzyl,

$R^2$ Alkyl oder Alkenyl

$R^3$ Alkyl, Cycloalkyl oder gegebenenfalls substituiertes Benzyl bedeuten, wobei die asymmetrischen Zentren des Moleküls $C_1$ und $C_2$

a) RR oder SS oder

b)RS oder SR

konfiguriert sind, die eine gute fungizide und pflanzenwachstumsregulierende Wirkung haben.

Mischungen, die 80 - 95 Gew.-% eines Diastereomers der Formel I a gemäß Anspruch 1 und 20 - 5 Gew.-% des entsprechenden Diastereomers der Formel I b enthalten, sowie Mischungen, die 80 - 95 Gew.-% eines Diastereomers der Formel I b gemäß Anspruch 1 und 20 - 5 Gew.-% des entsprechenden Diastereomers der Formel I a enthalten, sind ebenfalls gut wirksam.

Weiterhin wurde gefunden, daß die neuen Diastereomeren Triazolylglykolether der Formel I a,

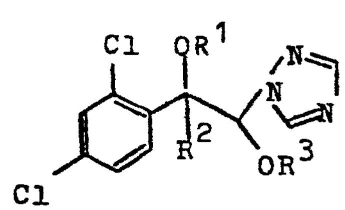

I a

Sws/Vz

in der $R^2$ und $R^3$ die obengenannten Bedeutungen haben und $R^1$ Wasserstoff bedeutet und $C_1$ und $C_2$ SS oder RR konfiguriert sind, mittels diastereoselektiver Grignardreaktion aus den Ketonen der Formel II

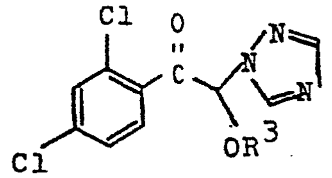 II

erhalten werden und die so erhaltenen erythro-Alkoholverbindungen gegebenenfalls zu Ethern der Formel I a

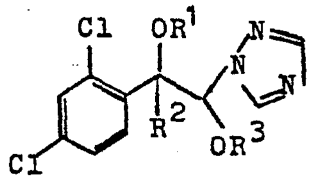 I a

in der $R^2$ und $R^3$ die obengenannten Bedeutungen haben und $R^1$ die obengenannten Bedeutungen außer Wasserstoff hat, wobei die asymmetrischen Kohlenstoffe $C_1$ und $C_2$ RR oder SS konfiguriert sind, umgesetzt werden. Die diastereoselektive Grignardreaktion erfolgt vorzugsweise, indem eine Lösung des Ketons II vorgelegt und zu dieser Lösung die metallorganische Verbindung $R^2$ MgX (X ist Halogen) langsam zugesetzt wird.

Es wurde ferner gefunden, daß die neuen Diastereomeren Triazolylglykolether der Formel I b

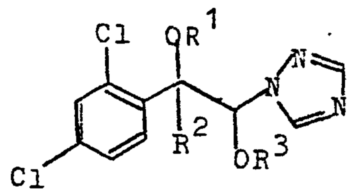 I b

in der $R^2$ und $R^3$ die obengenannten Bedeutungen haben und $R^1$ Wasserstoff bedeutet und $C_1$ und $C_2$ SR oder RS konfiguriert sind, mittels diastereoselektiver Grignardreaktion aus den Ketonen der Formel

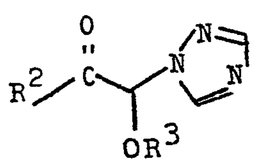 III,

in der $R^2$ und $R^3$ die obengenannten Bedeutungen haben, erhalten werden und die so erhaltenen threo-Alkoholverbindungen gegebenenfalls zu Ethern der Formel I b

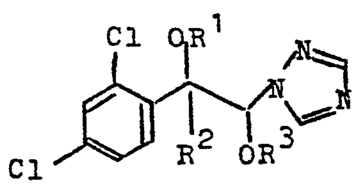 I b,

in der $R^1$, $R^2$ und $R^3$ die obengenannten Bedeutungen, außer Wasserstoff für $R^1$, haben, wobei die asymmetrischen Kohlenstoffe $C_1$ und $C_2$ RS oder SR konfiguriert sind, umgesetzt werden. Die diastereoselektive Grignardreaktion erfolgt vorzugsweise, indem eine Lösung des Ketons III vorgelegt und zu dieser Lösung die metallorganische Verbindung

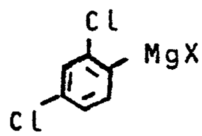

(X ist Halogen) langsam zugesetzt wird.

In der Formel I steht $R^1$ vorzugsweise für Wasserstoff, für einen Alkylrest mit 1 - 6 C-Atomen wie Methyl, Ethyl, n-Propyl, iso-Propyl, n-Butyl, iso-Butyl, 3-Methylbutyl, n-Pentyl oder einen gegebenenfalls durch Halogen, z.B. Chlor, oder $C_1$-$C_4$-Alkyl oder Trifluormethyl substituierten Benzylrest wie Benzyl, 4-Chlorbenzyl, 2,4-Dichlorbenzyl, 4-Fluorbenzyl, 4--Methylbenzyl, 4-$CF_3$-Benzyl oder einen Alkenylrest mit 2 - 4 C-Atomen wie Allyl, Crotyl oder für Propargyl.

$R^2$ steht vorzugsweise für einen Alkylrest mit 1 - 4 C-Atomen wie Methyl, Ethyl, n-Propyl oder für einen Alkenylrest mit 2 - 3 C-Atomen wie Vinyl oder Allyl.

$R^3$ steht vorzugsweise für einen Alkylrest mit 1 - 6 C-Atomen wie Methyl, Ethyl, n-Propyl, iso-Propyl, n-Butyl, iso-Butyl, 3-Methylbutyl, n-Pentyl oder Cyclohexyl, Methylcyclohexyl oder einen gegebenenfalls durch Halogen, z.B. Chlor, substituierten Benzylrest wie Benzyl, 4-Chlorbenzyl, 2,4-Dichlorbenzyl, 4-Fluorbenzyl.

Die neuen erythro-Triazolylglykolether der Formel I a

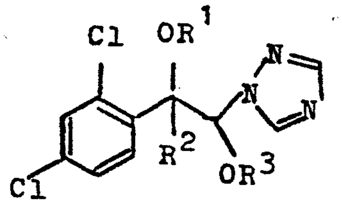 I a,

in der $R^1$ Alkyl, Alkenyl, Alkinyl, gegebenenfalls substituiertes Benzyl bedeuten und $R^2$ und $R^3$ die obengenannten Bedeutungen haben, wobei $C_1$ und $C_2$ RR und SS konfiguriert sind, werden erhalten durch Substitution an der OH-Gruppe der entsprechenden Alkohole

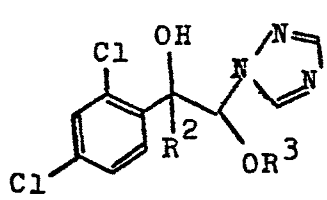 I a

der Formel I a, in der $R^2$ und $R^3$ die obengenannten Bedeutungen haben, wobei $C_1$ und $C_2$ RR und SS konfiguriert sind, mit den Halogenverbindungen $R^1X$, wobei X für Cl, Br, J steht, in Gegenwart eines Säurefängers, z.B. Kalium-tertiär-Butylat oder NaH in einem Lösungsmittel, z.B. Dimethylformaldehyd oder Dimethylsulfoxid (DMF, DMSO). Die Erythro-Konfiguration bleibt bei dieser Reaktion erhalten.

Die Erythro-Alkohole I a werden bevorzugt aus den Ketonen der Formel II hergestellt, in der $R^3$ die obengenannten Bedeutungen hat,

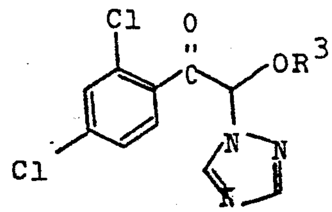 II,

indem man die Ketone II in einem organischen Lösungsmittel, beispielsweise einem Ether, z.B. Di-n-Butylether, Methyl-tert.-butylether, Diethylether oder einem aromatischen Kohlenwasserstoff, z.B. Toluol oder Tetrahydrofuran (THF), löst, diese Lösung vorlegt und die Suspension einer Grignardverbindung der Formel $R^2MgX$, in der $R^2$ die oben angegebenen Bedeutungen hat und X für Cl, Br, J steht, in THF oder einem Ether, beispielsweise Diethylether, Di-n-Butylether, Methyl-tert.-butylether, bei einer Temperatur von -10 bis +40°C, beispielsweise bei 0°C (Eiskühlung) langsam zu der vorgelegten Lösung zusetzt, z.B. zutropft. Dabei wird überraschend bevorzugt das erythro-Diastereomere I a gebildet. Das in DE 2 926 280 beschriebene Verfahren dagegen führt zu unerwünschten erythro/threo-Gemischen. Die Ausgangsverbindungen der Formel II sind zum Teil in DE 29 26 280 beschrieben und können nach den dort gegebenen Reaktionsvorschriften hergestellt werden.

Die neuen threo-Triazolylglykolether der Formel I b

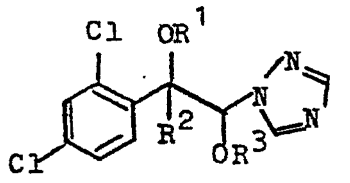 I b,

in der $R^1$, $R^2$ und $R^3$ die obengenannten Bedeutungen haben, außer Wasserstoff für $R^1$, und $C_1$ und $C_2$ RS und SR konfiguriert sind, werden erhalten durch Substitution an der OH-Gruppe der entsprechenden Alkohole der Formel I b

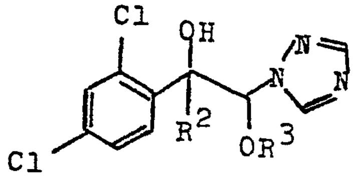,

in der $R^2$ und $R^3$ die obengenannten Bedeutungen haben und $C_1$ und $C_2$ RS und SR konfiguriert sind, mit Halogenverbindungen $R^1X$, wobei X für Cl, Br, J steht, in Gegenwart eines Säurefängers, z.B. Kalium-tertiär-Butylat oder NaH in einem Lösungsmittel, z.B. Dimethylformaldehyd oder Dimethylsulfoxid (DMF, DMSO). Die threo-Konfiguration bleibt bei dieser Reaktion erhalten.

Die threo-Alkohole der Formel I b werden bevorzugt aus Ketonen der Formel III hergestellt

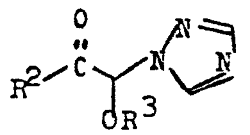
III,

in der $R^2$ und $R^3$ die obengenannten Bedeutungen haben, indem man die Ketone III in einem organischen Lösungsmittel, beispielsweise einem Ether, z.B. Di-n-butylether, Methyl-tert.-Butylether, Diethylether, oder einem aromatischen Kohlenwasserstoff, z.B. Toluol, oder Tetrahydrofuran (THF), löst, diese Lösung vorlegt und die Suspension einer Grignardverbindung der Formel

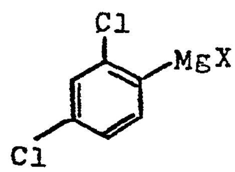
IV,

in der X Br oder J bedeutet in THF oder einem Ether, beispielsweise Diethylether, Di-n-butylether, Methyl-tert.-Butylether, bei einer Temperatur von -10 bis +40°C, beispielsweise 0°C (Eiskühlung) langsam zu der vorgelegten Lösung zusetzt, z.B. zutropft. Dabei wird überraschend bevorzugt das threo-Diastereomere I b gebildet. Das in DE 2 926 280 beschriebene Verfahren führt zu unerwünschten erythro/threo-Gemischen.

Die Ausgangsverbindungen der Formel III sind zum Teil in DE 2 926 280 beschrieben und können nach den dort gegebenen Reaktionsvorschriften hergestellt werden.

Die folgenden Beispiele erläutern die Darstellung der erfindungsgemäßen Diastereomere der Formel I.

Beispiel 1

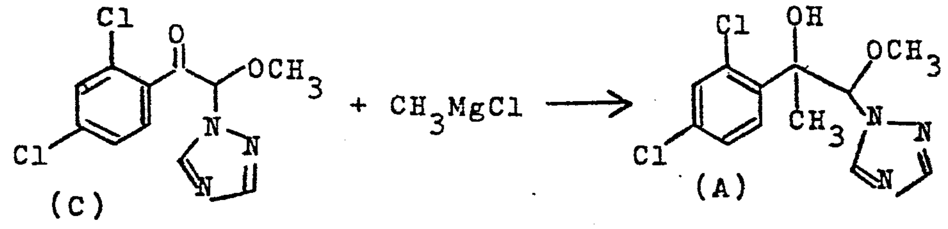


(C) (A)

Zu einer Lösung von 28,6 g Keton (C) in 200 ml THF werden zugetropft 134 ml einer 1,5 molaren Lösung von $CH_3MgCl$ in THF. Man erwärmt 2 h auf 50°C, läßt abkühlen, hydrolysiert mit gesättigter $NH_4Cl$-Lösung und extrahiert mit Ether. Man trocknet über $Na_2SO_4$ und engt ein. Das NMR-Spektrum des so erhaltenen Produkts zeigt zum größten Teil das erythro-Diastereomere (δ-Werte ($CDCl_3$) : $C\underline{H}_3COH$ : 1,9, $C\underline{H}OCH_3$ : 6,4) neben wenig Keton (C) und etwa 5 % (Gew.-%) threo-Diastereomer (δ-Werte ($CDCl_3$) : $CH_3COH$ : 1,4, $CHOCH_3$ : 6,5). Umkristallisation aus Toluol ergibt 21 g Alkohol A als reines Erythro-Diastereomer (Verbindung Nr. 1), Schmp. 138°C.

Beispiel 2

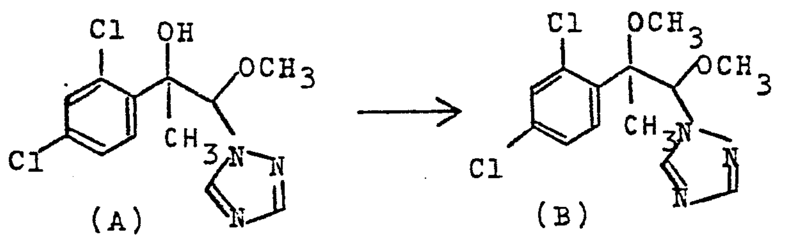

(A) (B)

Zu einer Lösung von 30 g Alkohol (A) und 17 g $CH_3J$ in 390 ml Diethylether und 110 ml DMSO werden portionsweise 3,6 g NaH (80 %ige Paraffinsuspension) zugegeben. Nach Beendigung der $H_2$-Entwicklung wird 1/2 h zum Rückfluß erwärmt. Man läßt abkühlen, tropft 1,2 l Wasser zu und extrahiert mit Diethylether. Die organische Phase wird mit wäßriger $Na_2S_2O_3$--Lösung und mit Wasser gewaschen, über $Na_2SO_4$ getrocknet und eingeengt. Umkristallisation aus Diisopropylether ergibt 21 g Methylether (B), Fp. 92°C (Verbindung Nr. 8).

Beispiel 3

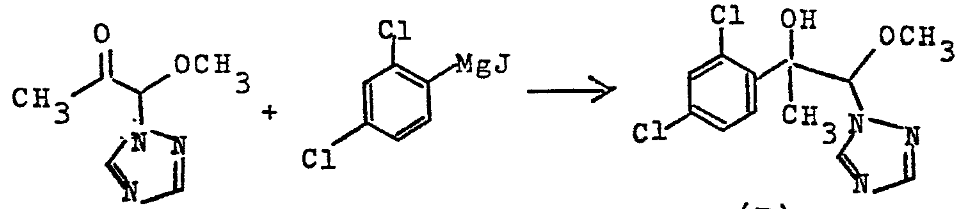

(D) (E)

Eine Grignardsuspension von 0,15 Mol 2,4-Dichlorphenylmagnesiumjodid in 150 ml Diethylether wird zu 0,1 Mol Keton D gelöst in 100 ml Diethylether zugetropft. Man rührt 5 h bei Raumtemperatur (20°C) und hydrolysiert mit 50 g Eis und 50 ml 25 %iger $NH_4Cl$-Lösung. Man extrahiert mit Ether, wäscht die organische Phase mit Wasser, trocknet über $Na_2SO_4$ und engt ein. Das Rohprodukt wird mit Diisopropylether verrührt, abgesaugt und mit Petrolether gewaschen. Man erhält 7,5 g Alkohol E - reines threo-Isomer, Fp. 134°C (Verbindung Nr. 14).

In entsprechender Weise werden die folgenden Verbindungen erhalten.

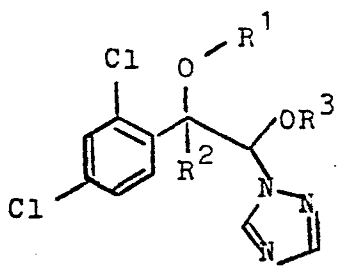

erythro-Diastereomere der Formel I a

| Nr. | $R^1$ | $R^2$ | $R^3$ | Fp/Sdp in °C |
|---|---|---|---|---|
| 1 | H | $CH_3$ | $CH_3$ | 138° |
| 2 | H | $C_2H_5$ | $CH_3$ | 130° |
| 3 | H | $CH_3$ | $C_2H_5$ | 147° |
| 4 | H | $CH_3$ | n-$C_3H_7$ | 97° |
| 5 | H | $CH_3$ | n-$C_4H_9$ | |
| 6 | H | $CH_3$ | Isobutyl | |
| 7 | H | $CH_3$ | n-Pentyl | 105° |
| 8 | $CH_3$ | $CH_3$ | $CH_3$ | 92° |
| 9 | $C_2H_5$ | $CH_3$ | $CH_3$ | 90° |
| 10 | Allyl | $CH_3$ | $CH_3$ | 58° |
| 11 | 2,4-$Cl_2$-Benzyl | $CH_3$ | $CH_3$ | 141° |
| 12 | $CH_3$ | $CH_3$ | n-Butyl | Harz |
| 13 | Propargyl | $CH_3$ | $C_2H_5$ | 114° |
| 14 | Propargyl | $CH_3$ | $CH_3$ | 120° |
| 15 | H | Vinyl | $CH_3$ | 141° |
| 16 | H | $CH_3$ | Benzyl | 162° |
| 17 | H | $CH_3$ | $CH_2$-Cyclohexyl | Harz |
| 18 | Allyl | $CH_3$ | n-Propyl | 174°/0,4 mbar |
| 19 | $CH_3$ | $CH_3$ | $C_2H_5$ | 64° |
| 20 | $CH_3$ | $CH_3$ | iso-Pentyl | 86° |

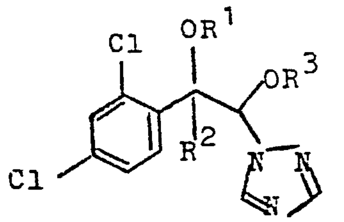

threo-Diastereomere der Formel I b

| Nr. | $R^1$ | $R^2$ | $R^3$ | Fp/Sdp in °C |
|---|---|---|---|---|
| 1.1 | $CH_3$ | $CH_3$ | n-Butyl | 160-163°/0,3 mbar |
| 1.2 | H | $CH_3$ | n-Propyl | 130° |
| 1.3 | H | $CH_3$ | $C_2H_5$ | 103° |
| 1.4 | H | $CH_3$ | $CH_3$ | 134° |
| 1.5 | $CH_3$ | $CH_3$ | $C_2H_5$ | 70° |
| 1.6 | H | $CH_3$ | n-Butyl | 96° |
| 1.7 | H | $CH_3$ | $CH_2$-Cyclohexyl | Harz |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten,
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit

Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 14 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid

an 1 Mol Ricinusöl besteht. Durch Ausgießen und feines Verteilen der Lösung in Wasser erhält man eine wäßrige Dispersion.

III. 20 Gew.-Teile der Verbindung Nr. 17 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 4 werden in einer Mischung gelöst, die aus 25 Gew.-Teilen Cyclohexanol, 65 Gew.-Teilen einer Mineralölfraktion vom Siedepunkt 210 bis 280$^0$C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 12 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 19 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 19 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 6 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 20 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines

paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,

2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol.

Anwendungsbeispiel 1

Wirksamkeit gegen Weizenmehltau

Blätter von in Töpfen gewachsenen Weizenkeimlingen der Sorte Frühgold werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Bewertung:

0 = kein Pilzbefall, abgestuft bis
5 = Totalbefall

A = leichter Blattschaden
B = mittlerer Blattschaden
C = starker Blattschaden

| Wirkstoff | Befall der Blätter nach Behandlung mit ... %iger Wirkstoffbrühe 0,025 | 0,006 | 0,0015 |
|---|---|---|---|
| 1 | 0 | 0 | 2 |
| 1.4 | 1A | 3 | 4 |
| 3 | 0 | 0 | 0 |
| 1.3 | 3B | 3-4 | 4 |
| 4 | 0 | 0 | 0 |
| 1.2 | 2 | 2 | 3 |
| 12 | 0 | 0 | 2 |
| 1.1 | 1 | 2 | 3-4 |
| 19 | 0A | 0A | 2 |
| 1.5 | 3 | 3 | 3-4 |
| Unbehandelt | | 5 | |

Anwendungsbeispiel 2

Wirksamkeit gegen Gurkenmehltau (kurativ)

Junge Gurkenpflanzen der Sorte "Chinesische Schlange" werden im Zweiblattstadium mit einer wäßrigen Konidiensuspension des Gurkenmehltaus (Erysiphe cichoracearum und Sphaerotheca fuliginea) besprüht. Nach 3 Tagen werden diese Pflanzen mit wäßriger Spritzbrühe, die 80 % Wirkstoff und 20 % Emulgiermittel in der Trockensubstanz enthält, bis zur Tropfnässe besprüht und im Gewächshaus bei Temperaturen zwischen 20 und 22°C und 70-80 % Luftfeuchtigkeit aufgestellt. 21 Tage nach der Wirkstoffapplikation wird das Ausmaß des Pilzbefalls ermittelt.

Bewertung:

0 = kein Pilzbefall, abgestuft bis
5 = Totalbefall

| Wirkstoff | Befall der Blätter nach Behandlung mit ... %iger Wirkstoffbrühe | |
|---|---|---|
| | 0,0125 | 0,006 |
| 1 | 0 | 0 |
| 1.4 | 1 | 2 |
| 3 | 0 | 0 |
| 1.3 | 3-4 | 4 |
| 12 | 0 | 0 |
| 1.1 | 1 | 2 |
| 19 | 0 | 1 |
| 1.5 | 3 | 3-4 |
| Unbehandelt | 5 | |

Anwendungsbeispiel 3

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

Bewertung:

0 = kein Pilzbefall, abgestuft bis
5 = Totalbefall

A = leichter Blattschaden
B = mittlerer Blattschaden
C = starker Blattschaden

| Wirkstoff | Befall der Blätter nach Behandlung mit 0,025%iger Wirkstoffbrühe |
|---|---|
| 1 | 1 |
| 1.4 | 3-4 |
| 3 | 2 |
| 1.3 | 3-4A |
| 4 | 0 |
| 1.2 | 3-4 |
| 12 | 0 |
| 1.1 | 2 |
| 19 | 0A |
| 1.5 | 3-4 |
| Unbehandelt | 5 |

Anwendungsbeispiel 4

Wirksamkeit gegen Apfelschorf

Junge Blätter von in Töpfen gewachsenen Apfelsämlingen der Sorte "Golden Delicious" werden mit wäßriger Spritzbrühe, die 80 % Wirkstoff und 20 % Emulgiermittel in der Trockensubstanz enthält, tropfnaß gespritzt. Nach dem Antrocknen des Spritzbelages werden die Versuchspflanzen mit einer Sporensuspension des Apfelschorfs (Venturia inaequalis) besprüht. Anschließend werden die inoculierten Pflanzen in einer Klimakammer bei 20 bis 22°C und 95 % relativer Luftfeuchtigkeit für 10 Tage aufgestellt. Dann wir das Ausmaß der Pilzentwicklung auf den Blättern ermittelt.

Bewertung:

0 = kein Pilzbefall, abgestuft bis
5 = Totalbefall

| Wirkstoff | Befall der Blätter nach Behandlung mit 0,0075%iger Wirkstoffbrühe |
|---|---|
| 4 | 0 |
| 6 | 0 |
| 20 | 1 |
| 1.2 | 1 |
| Unbehandelt | 4-5 |

Anwendungsbeispiel 5

Wirksamkeit gegen Botrytis cinerea an Paprika

Paprikasämlinge der Sorte "Neusiedler Ideal Elite" werden, nachdem sich 4-5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22-24°C in einer Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Bewertung:

0 = kein Pilzbefall, abgestuft bis
5 = Totalbefall

| Wirkstoff | Befall der Blätter nach Behandlung mit 0,05%iger Wirkstoffbrühe |
|---|---|
| 1 | 0 |
| 1.4 | 4 |
| 3 | 1 |
| 4 | 0 |
| 12 | 1 |
| 1.1 | 4 |
| 19 | 1 |
| Unbehandelt | 5 |

Patentansprüche

1. Diastereomere der Formel

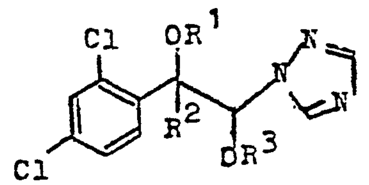 I,

in der

$R^1$ für Wasserstoff, Alkyl, Alkenyl, Alkinyl und gegebenenfalls substituiertes Benzyl,

$R^2$ für Alkyl und Alkenyl,

$R^3$ für Alkyl, Cycloalkyl und gegebenenfalls substituiertes Benzyl steht, wobei die asymmetrischen Zentren des Moleküls

a) RR oder SS oder

b) RS oder SR

konfiguriert sind.

2. Mischungen enthaltend 80 - 95 Gew.-% eines Diastereomers der Formel I a gemäß Anspruch 1 und 20 - 5 Gew.-% des entsprechenden Diastereomers der Formel I b.

3. Verfahren zur Herstellung der Diastereomere I a nach Anspruch 1, dadurch gekennzeichnet, daß man ein Keton der Formel

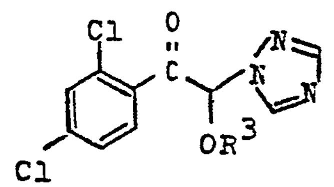 II,

in der $R^3$ die obengenannten Bedeutungen hat, in einer diastereoselektiven Grignardreaktion mit einer Verbindung der Formel $R^2MgX$ umsetzt, in der $R^2$ die im Anspruch 1 aufgeführten Bedeutungen hat und X Cl, Br oder J bedeutet und die so erhaltene Alkoholverbindung der Formel I a

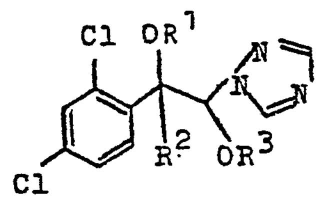 I a,

in der $R^2$ und $R^3$ die obengenannte Bedeutungen haben, $R^1$ Wasserstoff bedeutet und $C_1$ und $C_2$ RR oder SS konfiguriert sind,

gegebenenfalls zu den Ethern der Formel I a, in der $R^1$ die im Anspruch 1 genannten Bedeutungen außer Wasserstoff hat, umsetzt.

4. Verfahren zur Herstellung der Diastereomere I b nach Anspruch 1, dadurch gekennzeichnet, daß man ein Keton der Formel

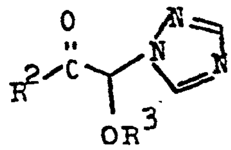 III,

in der $R^2$ und $R^3$ die in Anspruch 1 genannten Bedeutungen haben in einer diastereoselektiven Grignardreaktion mit einer Verbindung der Formel

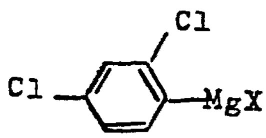 IV,

in der X Cl, Br oder J bedeutet, umsetzt und die so erhaltene Alkoholverbindung der Formel I b

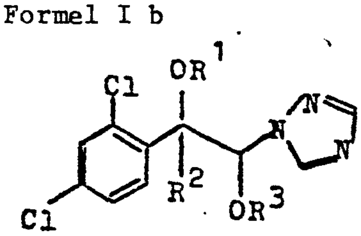 I b,

in der $R^2$ und $R^3$ die obengenannten Bedeutungen haben, $R^1$ Wasserstoff bedeutet und $C_1$ und $C_2$ RS oder SR konfiguriert sind, gegebenenfalls zu den Ethern der Formel I b, in der $R^1$ die im Anspruch 1 genannten Bedeutungen außer Wasserstoff hat, umsetzt.

5. Fungizides Mittel, gekennzeichnet durch einen Gehalt an einem üblichen Trägerstoff und einem weitgehend reinen Diastereomeren der gemäß Anspruch 8.

6. Erythro-Diastereomere der Verbindungen

1-(2,4-Dichlorphenyl)-1-hydroxy-2-methoxy-1-methyl-2-(1,2,4-triazol-1-yl)-ethan,

1-(2,4-Dichlorphenyl)-1-hydroxy-1-methyl-2-n-propoxy-2-(1,2,4-triazol-1-yl)-ethan,

2-n-Butoxy-1-(2,4-dichlorphenyl)-1-hydroxy-1-methyl-2-(1,2,4-triazol-1-yl)-ethan,

1-(2,4-Dichlorphenyl)-1-hydroxy-1-methyl-2-(3-methylbutoxy)-2-(1,2,4-triazol-1-yl)-ethan,

1-(2,4-Dichlorphenyl)-1-hydroxy-2-methoxy-2-(1,2,4-triazol-1-yl)-1-vinyl-ethan.

7. Verfahren zur Bekämpfung von Pilzen, <u>dadurch gekennzeichnet</u>, daß man die Pilze oder die von Pilzbefall bedrohten Pflanzen, Materialien, Saatgüter oder den Boden mit einer fungizid wirksamen Menge eines Diastereomeren gemäß Anspruch 8 behandelt.

8. Diastereomer der Formel

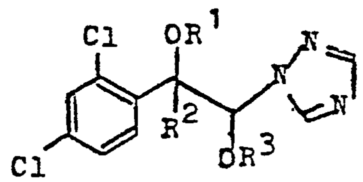

I,

in der

$R^1$ für Wasserstoff, Alkyl, Alkenyl, Alkinyl und gegebenenfalls substituiertes Benzyl,

$R^2$ für Alkyl und Alkenyl,

$R^3$ für Alkyl, Cycloalkyl und gegebenenfalls substituiertes Benzyl steht, wobei die asymmetrischen Zentren des Moleküls RR oder SS konfiguriert sind,

hergestellt nach dem Verfahren gemäß Anspruch 3.

9. Diastereomer der Formel

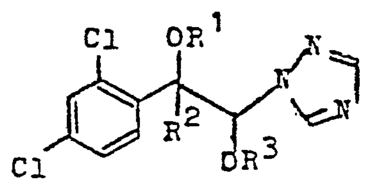

I,

in der

$R^1$ für Wasserstoff, Alkyl, Alkenyl, Alkinyl und gegebenenfalls substituiertes Benzyl,

$R^2$ für Alkyl und Alkenyl,

$R^3$ für Alkyl, cycloalkyl und gegebenenfalls substituiertes Benzyl steht, wobei die asymmetrischen Zentren des Moleküls RS oder SR konfiguriert sind,

hergestellt nach dem Verfahren gemäß Anspruch 4.